# EUROPEAN PATENT APPLICATION

(11) **EP 2 671 872 A1**
(43) Date of publication of application: **11.12.2013**
(21) Application number: 12170914.1
(22) Date of filing: 05.06.2012
(51) Int. Cl.: C07D 213/53, A61K 31/4402, A61P 25/00

(54) **Fluorinated pyridylacetylene derivatives as tracers**

(71) Applicant: Eidgenössische Technische Hochschule Zürich (ETH), 8092 Zürich (CH); Universität Zürich, 8006 Zürich (CH)
(72) Inventor: Milicevic Sephton, Selena, 8049 Zürich (CH); Ametamey, Simon Mensah, 8045 Zürich (CH); Kraemer, Stefanie, 8049 Zürich (CH); Schibli, Roger, 5400 Baden (CH); Mu, Linjing, 5600 Lenzburg (CH)
(74) Representative: Schmauder & Partner AG Patent- & Markenanwälte VSP

(57) **Abstract**

The invention relates to a novel compound of formula (I) 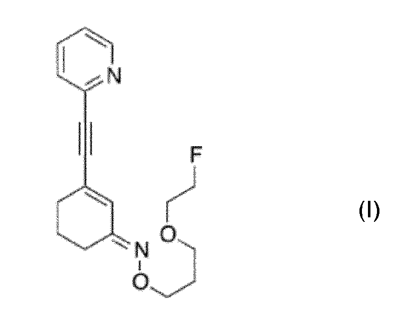 wherein F is ¹⁹F or ¹⁸F, in free base or acid addition salt form. In its radioactive form with ¹⁸F, the compound is useuful as a marker for radioimaging of brain and peripheral nervous system tissues or other tissues containing mGlu5 receptors *in vitro* or *in vivo*.

## Description

### Field of the Invention

The present invention relates to novel fluorinated pyridylacetylene derivatives, their preparation, their use as radiotracers/markers and compositions containing them.

### Background of the Invention

Metabotropic glutamate receptor subtype 5 (mGluR5) is a seven transmembrane domain, G-protein coupled receptor belonging to group I of the metabotropic glutamate receptors which plays a role in several disorders of the central nervous system such as schizophrenia, depression, anxiety, Alzheimer and Parkinson's disease. It is considered an important future drug target, and much attention has been given to the development of an optimal positron emission tomography (PET) tracer for imaging of mGluR5 (S Milicevic Sephton et al., Am J Nucl Med Mol Imaging 2 (2012) 14-28, and references cited therein).

To date, several PET tracers for imaging mGluR5 have been developed, of which (E)-3-((6-methylpyridin-2-yl)ethynyl)cyclohex-e-enone-O-¹¹C-methyl oxime (abbreviated: [¹¹C]-ABP688) is the most successful clinically applied tracer. However, due to the short physical half-life (about 20 min) of carbon-11, the application of [¹¹C]-ABP688 is limited to facilities with on-site cyclotron.

Accordingly, several attempts were made to develop fluorine-18 labeled radiotracers for PET imaging of mGluR5, which benefits from the longer physical half-life of fluorine-18 which is around 110 min. To date, the two most successful ¹⁸F-labeled radiotracers for PET imaging of mGluR5 in humans are 3-fluoro-5-(2-(2-[¹⁸F](fluoromethyl)-thiazol-4-yl)-ethynyl benzonitrile (abbreviated: [¹⁸F]-SP203), developed by Pike and coworkers (FG Simeon et al., J Med Chem 50 (2007) 3256-3266), and 3-[¹⁸F]fluoro-5-(2-pyridinylethynyl)benzonitrile (abbreviated: [¹⁸F]-FPEB), developed by Merck but applied by Hamill and coworkers (TG Hamill et al., Synapse 56 (2005) 205-216). However, [¹⁸F]-SP203 undergoes significant defluorination in rodents and monkeys and, to a lesser extent, in humans, whereas [¹⁸F]-FPEB is typically obtained in low radiochemical yields.

(E)-3-(Pyridin-2-ylethynyl)-cyclohex-2-enone-O-2-(2-¹⁸F-fluoroethoxy)ethyl oxime (abbreviated [¹⁸F]FDEGPECO), developed by Ametamey and co-workers, was the first ¹⁸F-labeled pyridylacetylene derivative with acceptable imaging quality in preclinical tests with rats (Wanger-Baumann et al., Neuroimage 56 (2011) 984-991). The tracer was not further promoted as its mean residence time at the target site was relatively short.

More recently, Ametamey and coworkers have reported about (E)-3-(pyridin-2-ylethynyl)cyclohex-2-enone O-(2-(3-¹⁸F-fluoropropoxy)ethyl) oxime (abbreviated [¹⁸F]PSS223), which can be prepared conveniently by a one-step radiosynthesis (S Milicevic Sephton et al., loc. cit.). However the compound has not been applied in human subjects due to rapid defluorination *in vivo* in rats.

Accordingly, a need exists for new fluorinated compounds having high specificity for mGluR5, optimal *in vivo* kinetics, and being amenable to preparation of a fluorine-18 labeled form suitable as PET tracer.

### Summary of the Invention

According to one aspect of the invention, there is provided a compound of formula (I) 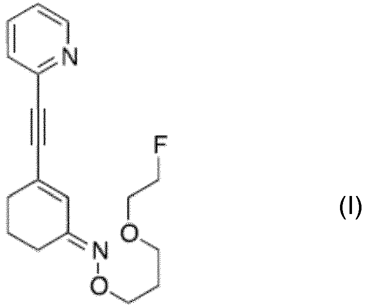 wherein F is ¹⁹F or ¹⁸F. This compound is named (E)-3-(pyridin-2-ylethynyl)-cyclohex-2-enone O-(3-(2-fluoroethoxy)propyl) oxime (henceforth abbreviated as: PSS232). The compound can be in free base or acid addition salt form.

The compound has high specificity for mGluR5. Moreover, it does not show significant defluorination *in vivo* in rats and is not significantly defluorinated by human and rat liver microsomes, respectively, and human S9 fraction of liver homogenate. This suggests negligible defluorination in human subjects.

Acid addition salts may be produced from the free bases in known manner, and vice-versa.

It will be understood that the non-radioactive compound wherein F is ¹⁹F cannot be used as a PET tracer. Nevertheless, the non-radioactive compound is useful in the general context of PET imaging of mGluR5, e.g. for comparative studies or competitive binding experiments where other mGluR5 ligands need to be displaced from the target.

In a particularly advantageous embodiment (claim 2) the compound is provided as radiolabelled compound wherein F is ¹⁸F. The radiolabelled compound, henceforth denoted as "radiolabelled agent of the invention" is generally useful as a PET tracer or for any method or device allowing detection of radioactive radiation, particularly beta⁺ and gamma radiation. It exhibits valuable properties as histopathological labeling agent, imaging agent and/or biomarker for the selective labeling of the metabotropic glutamate receptor subtype 5 (mGlu5 receptor).

The radiolabelled agent of the invention can be obtained in a single radiochemical step in reproducibly high (36%) radiochemical decay corrected yield. The total labeling time is less than 60 min after the end of bombardment and the radiolabelled agent is obtained in high specific activity and HPLC purity. The radiochemical step conveniently starts from a non-radioactive precursor that requires only six chemical preparation steps and can be prepared in reasonable quantities and stored at -20°C.

More particularly the radiolabelled agent of the invention is useful as marker for labeling the central and peripheral mGlu5 receptors *in vitro* or *in vivo.*

The radiolabelled agent of the invention is therefore useful, for instance for, but not limited to, determining the levels of receptor occupancy of a drug acting at the mGlu5 receptor, diagnostic and monitoring purposes for diseases resulting from or in an imbalance or dysfunction of mGlu5 receptors, and monitoring the effectiveness of pharmacotherapies of such diseases.

In accordance with the above, according to another aspect, the radiolabelled agent of the invention is provided for use as a marker for radioimaging (claim 3).

In a further aspect, the present invention provides a composition for labeling tissues containing mGlu5 receptors *in vivo* and *in vitro* comprising the radiolabelled agent of the invention (claim 6).

According to one embodiment, the agent is used for radioimaging of brain and peripheral nervous system structures (claims 4 and 7). According to another embodiment, the agent is used for radioimaging of structures containing beta cells (claim 5 and 8). Based on certain evidence indicating a higher expression of mGluR5 in beta cells than in surrounding cells, this embodiment could become of interest in diabetes diagnosis.

In still a further aspect, the present invention provides a method for labeling brain and peripheral nervous system tissues or any other tissues containing mGlu5 receptors *in vitro* or *in vivo,* which comprises contacting brain tissue with the radiolabelled agent of the invention (claim 9).

The present invention is generally useful for the imaging, detection, characterization of mGluR5 in any organ or tissue, including diagnosis, monitoring, and therapy assessment of tumor tissue.

The method of the invention may comprise a further step aimed at determining whether the radiolabelled agent of the invention labeled the target structure. Said further step may be effected by observing the target structure using positron emission tomography (PET) or single photon emission computed tomography (SPECT), or any device allowing detection of radioactive radiations.

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:
- Fig. 1: shows the synthesis of the precursor of PSS232;
- Fig. 2: shows the synthesis of ¹⁸F-labeled PSS232 and of unlabeled PSS232;
- Fig. 3: shows the binding affinity of PSS232 to rat mGluR5;
- Fig. 4: shows *in vitro* autoradiographies of rat brain slices, demonstrating that [¹⁸F]PSS232 selectively binds to rat mGluR5;
- Fig. 5: shows a PET scan of a Wistar rat brain, demonstrating that [¹⁸F]PSS232 accumulates in mGluR5-rich brain regions;
- Fig. 6: shows time activity curves (TAC) of [¹⁸F]PSS232 in the PET scans of Fig. 5;
- Fig. 7: shows TACs of [¹⁸F]PSS232 in comparison with [¹¹C]ABP688 and [¹⁸F]FDEGPECO;
- Fig. 8: shows [¹⁸F]PSS232 displacement *in vivo,* indicating that the accumulation of [¹⁸F]PSS232 in brain is mGluR5-specific;
- Fig. 9: shows the kinetic behavior of various mGluR5 radiotracers;
- Fig. 10: shows *ex vivo* brain distributions of [¹⁸F]PSS232, demonstrating a high specific accumulation of [¹⁸F]PSS232 in mGluR5-rich brain regions; and
- Fig. 11: shows PSS232 displacement by MMPEP as a function of MMPEP dose, indicating that PSS232 displacement by MMPEP is dose dependent.

### Detailed description of the invention

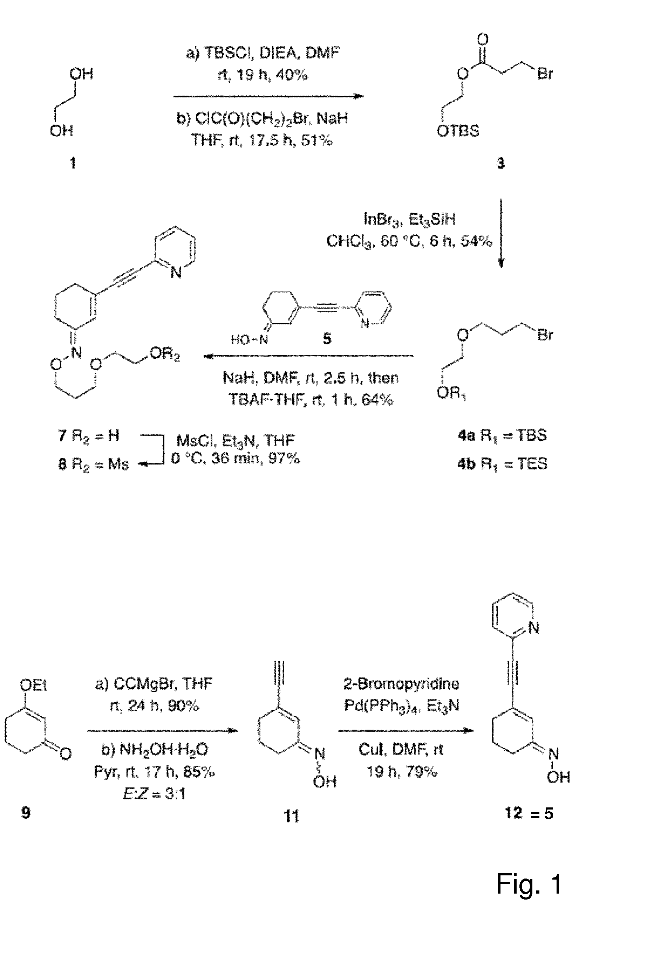

As shown in Fig. 1, preparation of the PSS232 precursor starts with ethylene glycol 1, which in four steps is converted to precursor **8.** It should be noted that compound **5,** which is added to compound **4a** or **4b** in the fifth reaction step, can be prepared in a known and established two-step synthesis from 3-ethoxy-2-cyclohexen-1-one (compound **9,** see lower part of Fig. 1). The precursor **8** can be stored at -20°C.

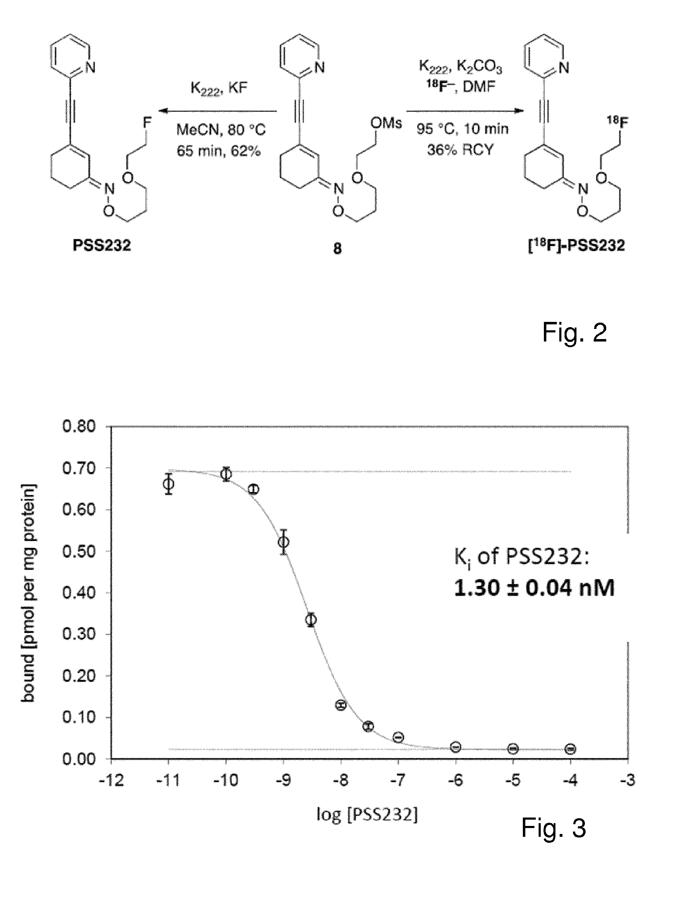

The actual synthesis of radiolabelled or unlabeled PSS232 starting from precursor **8** is shown in Fig. 2. Preparation of unlabeled PSS232 is carried out by reacting the precursor **8** with KF/kryptofix 2.2.2 complex in hot acetonitrile (80°C, 65 min), providing a yield of 62%. In contrast, preparation of radiolabelled [¹⁸F]-PSS232 is conducted by reacting the precursor **8** with K₂CO₃/kryptofix 2.2.2/¹⁸F complex in hot dimethylformamide (95°C, 10 min), providing a radiochemical yield of 36%. Similar radiochemical yields are obtained using DMSO or acetonitrile.

The following examples illustrate the invention.

### Example 1

The binding affinity of PSS232 to rat mGluR5 was studied in a displacement assay with rat brain membranes and 1.6 nM [³H]ABP688 (K_{D} 1.7 nM). Fig. 3 shows one out of three independent determinations of the half maximal inhibitory concentration (IC50), including the averages and standard deviations from three samples each. The sigmoidal red line is a fit to the data; the magenta upper horizontal line corresponds to the situation with no PSS232 added whereas the green lower horizontal line corresponds to unspecific binding at 100 µM MMPEP.

### Example 2

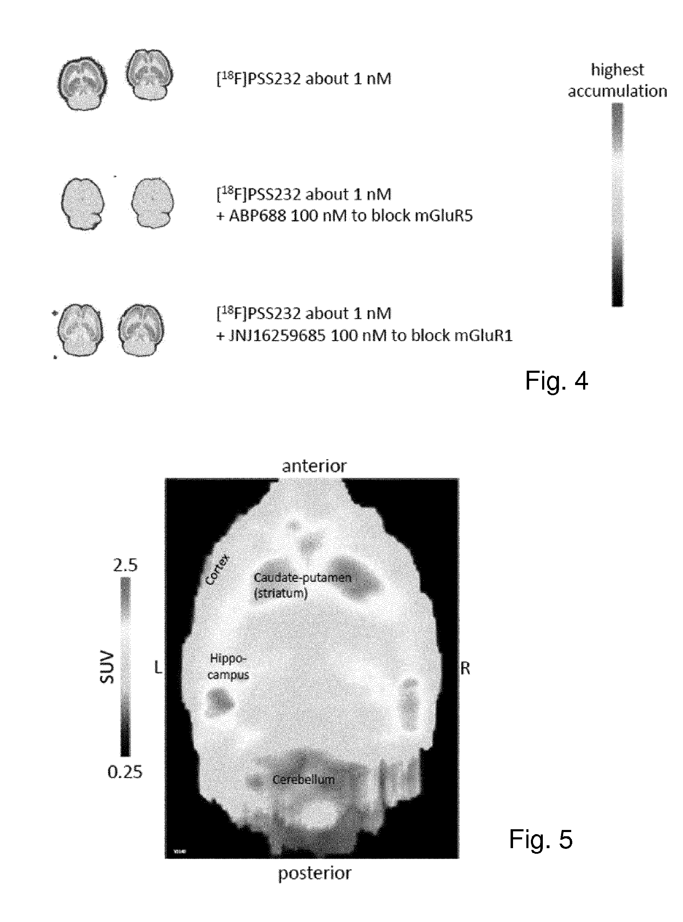

In Fig. 4 two rat brain slices (20 µm) each were incubated with about 1 nM [¹⁸F]PSS232 in the absence of any unlabeled compound (top) or with 100 nM of the specific mGluR5 ligand ABP688 (middle) or of mGluR1 ligand JNJ16259685 (bottom). A high, mGluR5-selective accumulation of [¹⁸F]PSS232 in mGluR5-rich domains was found. It should be noted that mGluR1 is the receptor with most similar amino acid sequence to mGluR5.

### Example 3

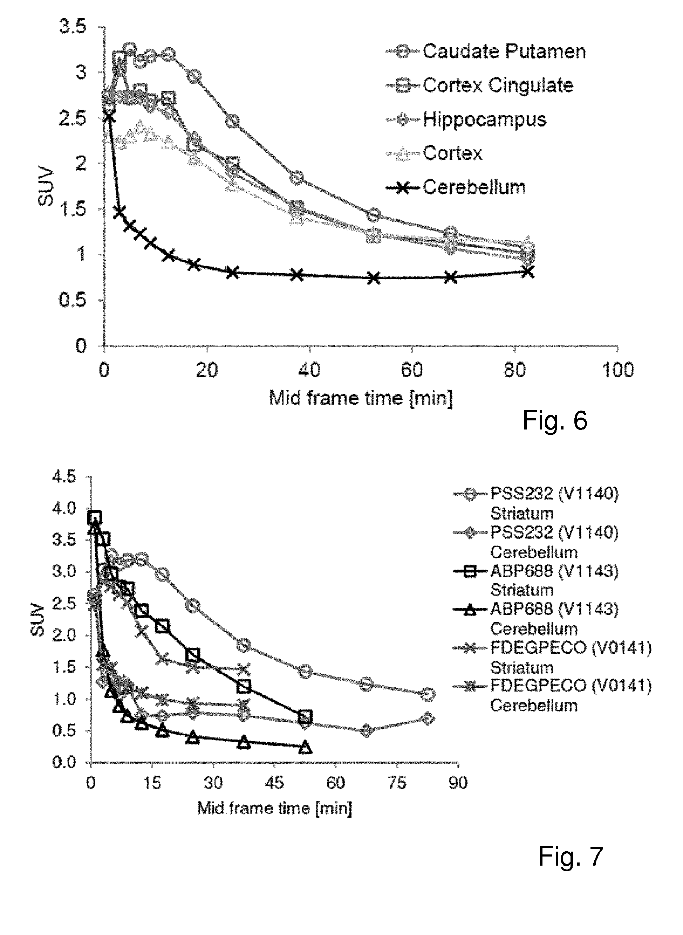

A male Wistar rat (423 g) was injected 23.1 MBq [¹⁸F]PSS232 into a tail vein and PET was acquired from 0 to 90 min post injection. Fig. 5 shows a PET image averaged 2-90 min post injection. Only brain is shown. SUV denotes standardized uptake value. Fig. 6 shows time-radioactivity curves of the PET scan with [¹⁸F]PSS232 shown in Fig. 5 in various brain regions.

### Example 4

PET scans analogous to those of Example 3 were recorded after administration of [¹⁸F]PSS232 (Fig. 7, red curves, circles and diamonds), [¹¹C]ABP688 (Fig. 7, black curves, squares and triangles) and [¹⁸F]FDEGPECO (Fig. 7, blue curves, crosses and crossed bars). Fig. 7 shows time-dependent SUVs of striatum (circles, squares, crosses, high mGluR5 expression) and cerebellum (diamonds, triangles, crossed bars, reference tissue, low mGluR5).

### Example 5

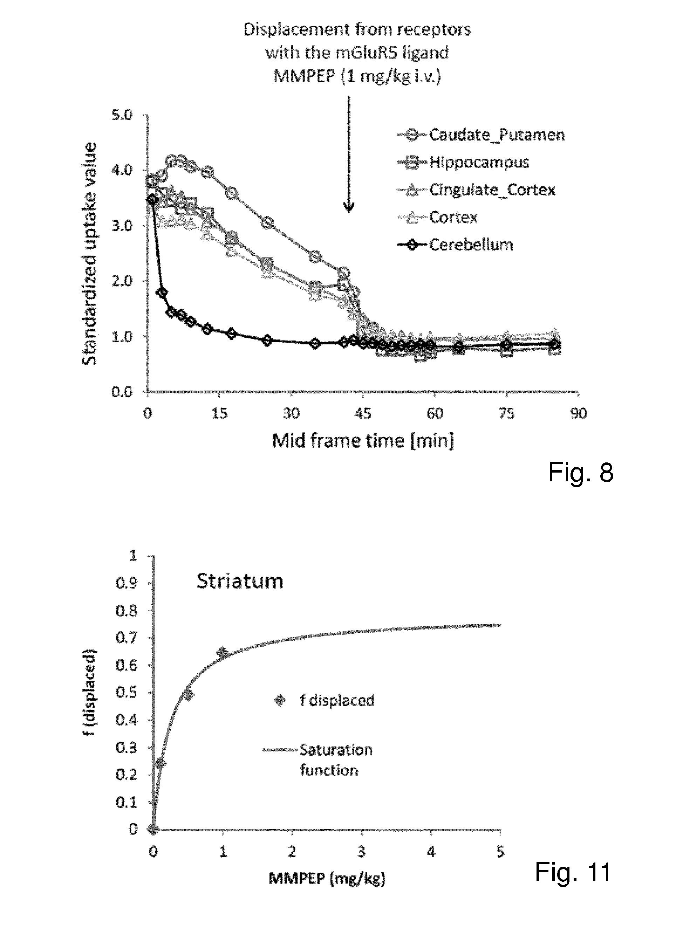

Fig. 8 shows SUV in various brain regions taken from a PET scan with [¹⁸F]PSS232. From 40 to 41 min following tracer injection and scan start a dose of MMPEP (1 mg/kg) was injected into a tail vein. This resulted in displacement of specific PSS232 binding, as evidenced by the sudden drop of SUVs.

### Example 6

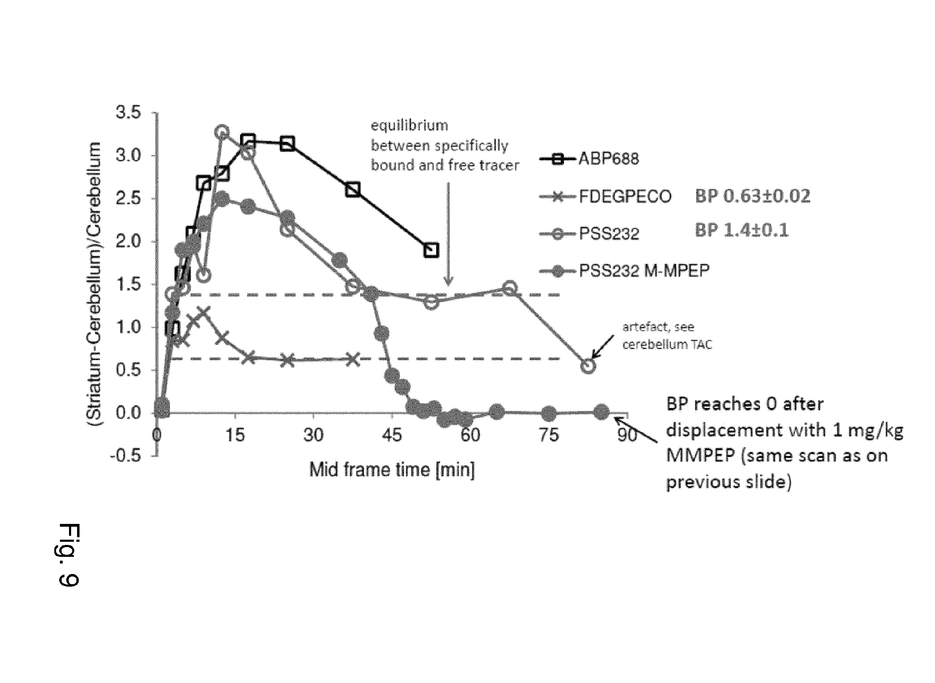

Fig. 9 illustrates the favorable kinetic behavior of PSS232. The ratio of specifically bound PSS232 in caudate putamen (striatum) to free ligand PSS232 (radioactivity in cerebellum is considered to originate from free ligand) becomes constant at about 40 min after injection, thus allowing the estimation of the binding potential (BP). This is an advantage over [¹¹C]ABP688 where the equilibrium is not reached within 60 min and the short physical half-life of ¹¹C (20 min) limits the scan duration.

### Example 7

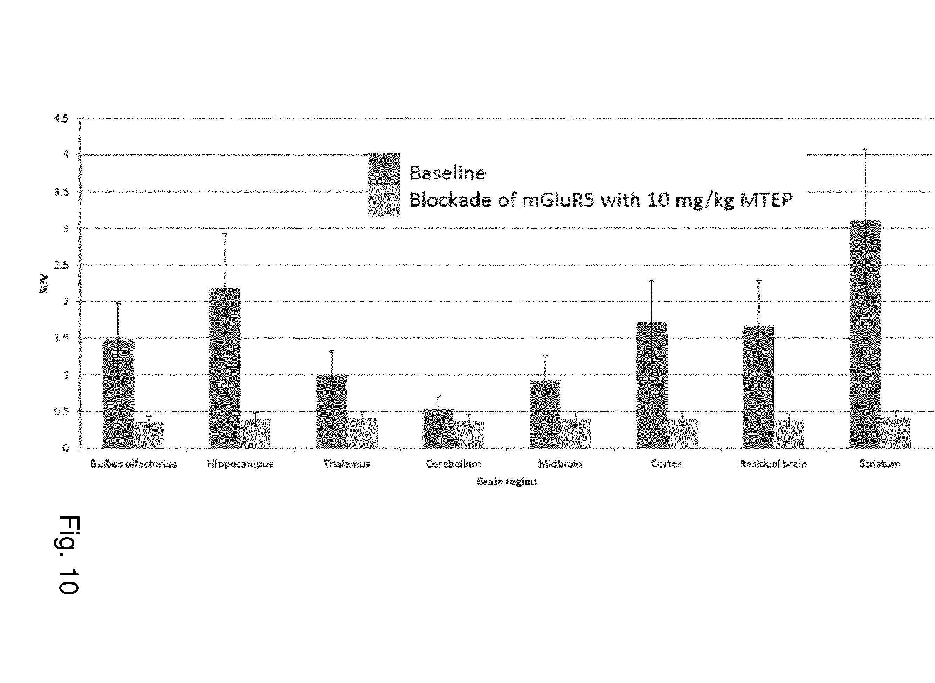

Rats were pre-injected (tail vein) with 10 mg/kg MTEP (a specific mGluR5 antagonist) before [¹⁸F]PSS232 injection (= blockade, see green, right bars in Fig. 10) or they were pre-injected with vehicle (= baseline, see blue, left, bars in Fig. 10). At 45 min after injection, the rats were sacrificed, brain regions removed and their radioactivity was measured in a gamma counter. Note that after MTEP pre-injection all brain regions have similar radioactivity as in cerebellum (corresponding to free ligand and possibly non-specific binding). Without MTEP blockade, a high specific binding was found in certain brain regions; the average specific binding in striatum was found to be 86.6 %.

### Example 8

Rats were pre-injected (tail vein) either with MMPEP (a specific mGluR5 antagonist) at various doses, or with vehicle before [¹⁸F]PSS232 injection. At 45 min after injections, rats were sacrificed, brain regions removed and their radioactivity was measured in a gamma counter, see data in Fig. 11. Receptor occupancy by [¹⁸F]PSS232 was dependent on the MMPEP dose and followed a typical saturation function (red fit line). This indicates that [¹⁸F]PSS232 is useful for receptor occupancy studies *in vivo.* From the fit to the data, the maximal specific binding of [¹⁸F]PSS232 in striatum was estimated as 78.5 % under these experimental conditions.

## Claims

1. A compound of formula (I) 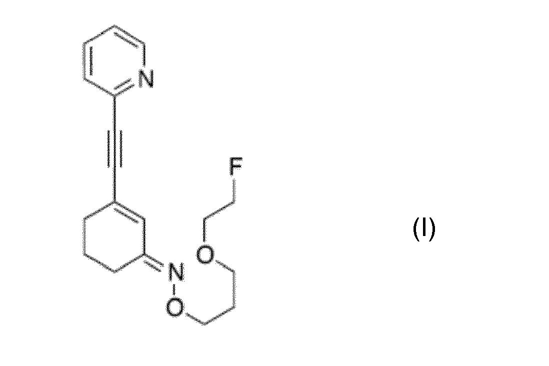 wherein F is ¹⁹F or ¹⁸F, in free base or acid addition salt form.

2. The compound according to claim 1, wherein F is ¹⁸F.

3. The compound according to claim 2, for use as a marker for radioimaging.

4. The compound according to claim 2, for use as a marker for radioimaging of brain and peripheral nervous system structures.

5. The compound according to claim 2, for use as a marker for radioimaging of structures containing beta cells.

6. A composition for labeling tissues containing mGlu5 receptors *in vivo* and *in vitro,* comprising the compound according to claim 2.

7. The composition according to claim 6, wherein said tissues are from brain or peripheral nervous system structures.

8. The composition according to claim 6, wherein said tissues are from structures containing beta cells.

9. A method for labeling brain and peripheral nervous system tissues or other tissues containing mGlu5 receptors in vitro or in vivo, which comprises contacting said tissues with the compound according to claim 2.
